# EUROPÄISCHE PATENTSCHRIFT

(11) **EP 0 941 097 B1**
(45) Veröffentlichungstag und Bekanntmachung des Hinweises auf die Patenterteilung: **02.05.2002**
(21) Anmeldenummer: 97950201.0
(22) Anmeldetag: 19.11.1997
(51) Int. Cl.: A61K 31/575

(54) **VERWENDUNG VON WIRKSTOFFMISCHUNGEN ZUR HERSTELLUNG VON HYPOCHOLESTERINÄMISCHEN MITTELN**
USE OF MIXTURES OF ACTIVE SUBSTANCES FOR THE PRODUCTION OF HYPOCHOLESTEREMIC AGENTS
UTILISATION DE MELANGES DE PRINCIPES ACTIFS POUR LA PRODUCTION D'AGENTS HYPOCHOLESTEROLEMIANTS

(30) Priorität: 28.11.1996 DE 19649286; 13.01.1997 DE 19700796
(43) Veröffentlichungstag der Anmeldung: 15.09.1999
(73) Patentinhaber: Cognis Deutschland GmbH & Co. KG, 40589 Düsseldorf (DE)
(72) Erfinder: WEITKEMPER, Norbert, D-51375 Leverkusen (DE); FABRY, Bernd, D-41352 Korschenbroich (DE)
(86) Internationale Anmeldenummer: EP9706447
(87) Internationale Veröffentlichungsnummer: WO9823275

(56) Entgegenhaltungen:
- CHEMICAL ABSTRACTS, vol. 79, no. 13, 1.Oktober 1973 Columbus, Ohio, US; abstract no. 77319, YASUI, AKIKO ET AL: "Synergistic effect of tocopherols on plasma cholesterol -lowering effect of vegetable sterols" XP002061647 & EIYO TO SHOKURYO (1973), 26(1), 27-32 CODEN: EISOAU, 1973,
- PATENT ABSTRACTS OF JAPAN vol. 007, no. 056 (C-155), 8.März 1983 & JP 57 206336 A (AJINOMOTO KK), 17.Dezember 1982,

## Beschreibung

### Gebiet der Erfindung

Die Erfindung betrifft die Verwendung von Mischungen aus Phytostanolestem und Tocopherolen zur Herstellung von Mitteln zur Verminderung des Cholesteringehaltes im Serum von Warmblütern.

### Stand der Technik

Unter hypochloesterinämischen Wirkstoffen werden Mittel verstanden, die zu einer Verminderung des Cholesteringehaltes im Serum von Warmblütern führen, ohne daß dadurch eine Hemmung oder Verringerung der Bildung von Cholesterin im Blut eintritt. Für diesen Zweck wurden bereits von Peterson et al. in **J.Nutrit. 50, 191 (1953)** Phytosterole, also pflanzliche Sterole, und deren Ester mit Fettsäuren vorgeschlagen. In die gleiche Richtung weisen auch die Patentschriften **US 3,089,939, US 3,203,862** sowie die deutsche Offenlegungsschrift **DE-OS 2035069** (Procter & Gamble). Die Wirkstoffe werden üblicherweise Brat- oder Speiseölen zugesetzt und dann über die Nahrung aufgenommen, wobei die Einsatzmengen jedoch in der Regel gering sind und üblicherweise unter 0,5 Gew.-% liegen, um zu verhindern, daß die Speiseöle eintrüben oder die Sterole bei Zusatz von Wasser ausgefällt werden. Für den Einsatz im Nahrungsmittelbereich, in Kosmetika, pharmazeutischen Zubereitungen und im Agrarsektor werden in der europäischen Patentanmeldung **EP-A1 0289636** (Ashai) lagerstabile Emulsionen der Sterolester in Zucker- oder Polyglycerinestem vorgeschlagen. Die Einarbeitung von Sitostanolestem zur Verminderung des Blutcholesteringehaltes in Margarine, Butter, Mayonnaise, Salatsaucen und dergleichen wird in der Europäischen Patentschrift **EP-B1 0594612** (Raision) vorgeschlagen.

**Chemical Abstracts, vol. 79, no. 13, (1973) abstract n. 77319** offenbart die Kombination eines Phytostenols aus Soyabohnen mit Tocopherolen, wobei eine Senkung des Plasmacholesterins gezeigt werden konnte.

**Patent Abstracts of Japan, vol. 007, no. 056, 1983 & JP 57 206336 A (1982)** referiert über ein zum Verzehr geeignetes pflanzliches Öl, das Vitamin E und ein pflanzliches Stenol, z.B. Sitosterol, enthält. Besagtes Öl soll den Anstieg von Cholesterin im Blut verhindern.

Von Nachteil ist jedoch, daß die Phytostanolester den Nahrungsmitteln üblicherweise nur in geringen Mengen zugesetzt werden können, da ansonsten die Gefahr besteht, daß sie den Geschmack und/oder die Konsistenz der Mittel beeinträchtigen. Zur nachhaltigen Beeinflussung des Cholesteringehaltes im Blut wäre jedoch die Aufnahme größerer Mengen Phytostanolester wünschenswert. Weiterhin verbesserungswürdig ist die Geschwindigkeit, mit der die Stoffe den Gehalt an Cholesterin im Serum vermindern. Die Aufgabe der Erfindung hat folglich darin bestanden, diesen Mängeln abzuhelfen.

### Beschreibung der Erfindung

Gegenstand der Erfindung ist die Verwendung von Wirkstoffmischungen zur Herstellung von hypocholesterinämischen Mitteln mit der Maßgabe, daß man
(a) Phytostanolester und
(b) Tocopherolen
einsetzt.

Überraschenderweise wurde gefunden, daß Tocopherole, die selbst über keine oder nur sehr geringe hypochloesterinämischen Eigenschaften verfügen, als Potenzierungsmittel für Phytostanolester wirken, d.h. die Abnahme des Cholesteringehaltes im Serum in Gegenwart von Phytostanolestem beschleunigen. In Gelatine verkapselt lassen sich sowohl die Phytostanolester als auch die Wirkstoffgemische problemlos oral einnehmen.

### Phytostanolester

Unter Phytostenolen (oder synonym Phytosterolen) sind pflanzliche Steroide zu verstehen, die nur am C-3 eine Hydroxylgruppe, sonst aber keine funktionellen Gruppen tragen. In der Regel besitzen die Phytosterole 27 bis 30 Kohlenstoffatome und eine Doppelbindung in 5/6, gegebenenfalls 7/8, 8/9 oder anderen Positionen. Durch Härtung werden aus den Phytostenolen die Phytostanole erhalten, welche nach Veresterung mit Fettsäuren die Komponente (a) bilden. Die Phytostanolkomponente kann sich von den Härtungsprodukten von Ergosterolen, Campesterolen, Stigmasterolen, Brassicasterolen, vorzugsweise Sitosterolen und insbesondere β-Sitostanolen ableiten. Die Säurekomponente der Ester kann auf Carbonsäuren der Formel **(I)** zurückgehen,

**R**^{**1**}**CO-OH (I)**

in der R¹CO für einen aliphatischen, linearen oder verzweigten Acylrest mit 2 bis 22 Kohlenstoffatomen und 0 und/oder 1, 2 oder 3 Doppelbindungen steht. Typische Beispiele sind Essigsäure, Propionsäure, Buttersäure, Valeriansäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Besonders bevorzugt ist der Einsatz von Estem des β-Sitostanols mit Fettsäuren mit 12 bis 18 Kohlenstoffatomen. Diese Ester können sowohl durch direkte Veresterung der Phytostanole mit den Fettsäuren oder aber durch Umesterung mit Fettsäureniedrigalkylestem oder Triglyceriden in Gegenwart geeigneter Katalysatoren, wie z.B. Natriumethylat oder speziell auch Enzymen hergestellt werden [vgl. **EP-A2 0195311** (Yoshikawa)]. Es ist ebenfalls möglich, zunächst die entsprechenden Phytostenolester herzustellen und diese anschließend unter Erhalt der Carbonylestergruppe zu härten.

### Tocopherole

Unter Tocopherolen werden in 2-Stellung mit 4,8,12-Trimethyltridecyl-Resten substituierte Chroman-6-ole (3,4-Dihydro-2-*H*-1benzopyran-6-ole) verstanden, die der Formel **(II)** folgen, 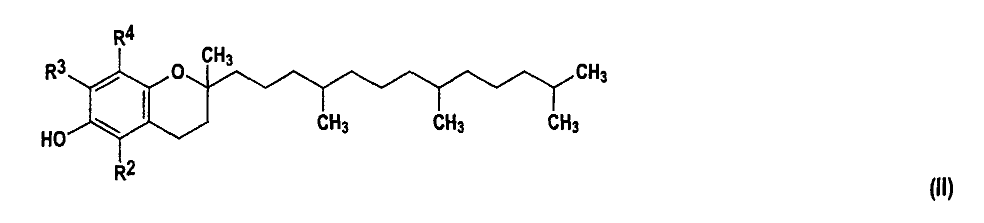 in der R², R³ und R⁴ unabhängig voneinander für Wasserstoff oder eine Methylgruppe stehen. Tocopherole zählen zu den Biochinonen, also polyprenylierten 1,4-Benzo- bzw. Naphthochinonen, deren Prenylketten mehr oder weniger stark gesättigt sind. Typische Beispiele für Tocopherole, die im Sinne der Erfindung als Komponente (b) in Betracht kommen, sind Ubichinone, Bovichinone, K-Vitamine und/oder Menachinone (2-Methyl-1,4-Naphthochinone). Man unterscheidet bei den Tocopherolen weiterhin α, β, γ-, δ- und ε-Tocopherole, wobei letztere noch über die ursprüngliche ungesättigte Prenylseitenkette verfügen, sowie α-Tocopherolchinon und -hydrochinon, bei denen das Pyran-Ringsystem geöffnet ist. Vorzugsweise wird als Komponente (b) α-Tocopherol (Vitamin E) der Formel **(II)** eingesetzt, bei der R², R³ und R⁴ für Methylgruppen stehen, oder Ester des α-Tocopherols mit Carbonsäuren mit 2 bis 22 Kohlenstoffatomen, wie beispielsweise α-Tocopherolacetat oder α-Tocopherolpalmitat.

### Chitosane

Als weitere Potenzierungsmittel (Komponente c1) können die Zubereitungen Chitosane enthalten. Hierbei handelt es sich um Biopolymere, die zur Gruppe der Hydrokolloide gezählt werden. Chemisch betrachtet handelt es sich um partiell deacetylierte Chitine unterschiedlichen Molekulargewichtes, die den folgenden - idealisierten - Monomerbaustein **(III)** enthalten:

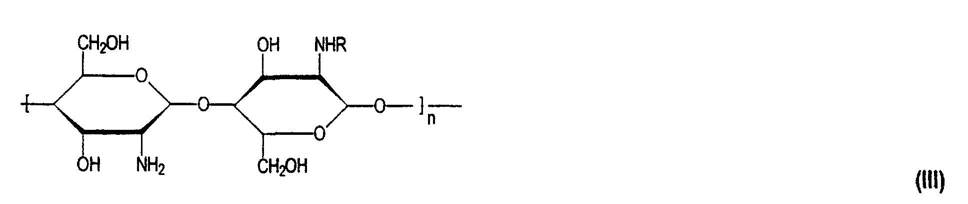

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332)**. Übersichten zu diesem Thema sind auch beispielsweise von B.Gesslein et al. in **HAPPI 27, 57 (1990),** O.Skaugrud in **Drug Cosm.lnd. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol.Chem. 177, 3589 (1976)** oder der französischen Patentanmeldung **FR-A 2701266** bekannt. In einer bevorzugten Ausführungsform der Erfindung wird ein Chitinabbauprodukt, wie es in der Internationalen Patentanmeldung **WO 96/16991** (Henkel) beschrieben wird, oder dessen Abbauprodukt mit Wasserstoffperoxid eingesetzt.

### Phytosterolsulfate

Als weitere Potenzierungsmittel (Komponente c2) können die Zubereitungen Phytosterolsulfate enthalten. Phytosterolsulfate stellen bekannte Stoffe dar, die beispielsweise durch Sulfatierung von Phytosterolen mit einem Komplex aus Schwefeltrioxid und Pyridin in Benzol hergestellt werden können [vgl. **J.Am.Chem.Soc. 63, 1259 (1941)**]. Typische Beispiele sind die Sulfate von Ergosterolen, Campesterolen, Stigmasterolen und Sitosterolen. Die Phytosterolsulfate können als Alkali- und/oder Erdalkalimetallsalze, als Ammonium-, Alkylammonium-, Alkanolammonium- und/oder Glucammoniumsalze vorliegen. In der Regel werden sie in Form ihrer Natriumsalze eingesetzt.

### (Desoxy)Ribonucleinsäuren

Als weitere Potenzierungsmittel (Komponente c3) können die Zubereitungen schließlich Ribo- bzw. Desoxyribonucleinsäuren enthalten. Unter (Desoxy)Ribonucleinsäuren (DNA bzw. RNA) werden hochmolekulare, fadenförmige Polynucleotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzelstrang-DNA bzw.-RNA. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton reichen. Im Sinne der Erfindung werden konzentrierte DNA bzw. RNA-Lösungen eingesetzt, die sich durch ein flüssig-kristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 40.000 bis 1.000.000 Dalton aufweisen.

### Gewerbliche Anwendbarkeit

Die Wirkstoffmischungen der Erfindung können die Phytostanolester und die Tocopherole im Gewichtsverhältnis 99:1 bis 1 : 99, vorzugsweise 90 : 10 bis 10 : 90, insbesondere 70 : 25 bis 25 : 75 und besonders bevorzugt 60 : 40 bis 40 : 60 enthalten, wobei allein sicherzustellen ist, daß mit der erfindungsgemäßen Verwendung eine zur Senkung des Cholesteringehaltes im Blut ausreichende Menge der Komponente (a) aufgenommen wird. In einer besonderen Ausführungsform der Erfindung werden die Wirkstoffmischungen in an sich bekannter Weise in Gelatine verkapselt, wobei man die Komponenten (a) und (b) jeweils in Mengen von 0,1 bis 50, vorzugsweise 1 bis 30, insbesondere 5 bis 25 und besonders bevorzugt 10 bis 15 Gew.-% - bezogen auf das Gewicht der Gelatinekapseln - einsetzt. Die Verkapselung der Phytostanolester in Gelatine, alleine oder in Abmischung mit den Potenzierungsmitteln, stellt dabei eine vorteilhafte Ausführungsform für die orale Aufnahme der Wirkstoffe dar. Der Anteil der weiteren Potenzierungsmittel (Komponente c) kann 1 bis 10 Gew.-% - bezogen auf die Wirkstoffmischungen - betragen.

Eine weitere Verabreichungsform der Wirkstoffmischungen sind Zäpfchen, die rektal oder vaginal eingeführt werden können, und als Supositoriengrundmasse ebenfalls Gelatine, gegebenenfalls in Kombination mit Glycerin, oder aber synthetische Fette bzw. Wachse, Polyethylenglycole oder natürliche Bestandteile wie z.B. Kakaobutter enthalten können. Daneben ist es möglich, die Mischungen in üblichen Nahrungsmitteln zu lösen bzw. zu dispergieren, als da beispielsweise sind: Salatöle, Dressings, Mayonnaisen, Margarinen, Butter, Fritierfette, Kakaoprodukte, Wurst und dergleichen.

### Beispiele

### Beispiele 1 bis 4, Vergleichsbeispiele V1 bis V4

Es wurden Gelatinekapseln (Gewicht ca. 1,5 g) mit einem Gehalt an β-Sitostanol- bzw. -stenolester und gegebenenfalls Tocopherol sowie 0,5 Gew.-% radioaktiv markiertem Cholesterin hergestellt. Zur Untersuchung der hypocholesterinämischen Wirkung ließ man männliche Ratten (Einzelgewicht ca. 200 g) über Nacht fasten. Am folgenden Tag wurde den Versuchstieren jeweils eine zerkleinerte Gelatinekapsel mit etwas kochsalzhaltigem Wasser über eine Magensonde eingeführt. Nach 3, 6, 12, 24 und 48 h wurde den Tieren Blut abgenommen und der Gehalt an radioaktivem Cholesterin bestimmt. Die Ergebnisse, die den Mittelwert der Messungen von 10 Versuchstieren darstellen, sind in Tabelle 1 zusammengefaßt. Die Angaben zur Abnahme der Radioaktivität verstehen sich jeweils in Bezug auf eine Blindgruppe von Versuchstieren, denen lediglich Gelatinekapseln mit einem Gehalt von 20 Gew.-% Vitamin E und einer entsprechenden Menge radioaktiv markiertem Cholesterin verabreicht worden war. Die Mischungen 1 bis 4 sind erfindungsgemäß, die Mischungen V1 bis V4 dienen dem Vergleich.

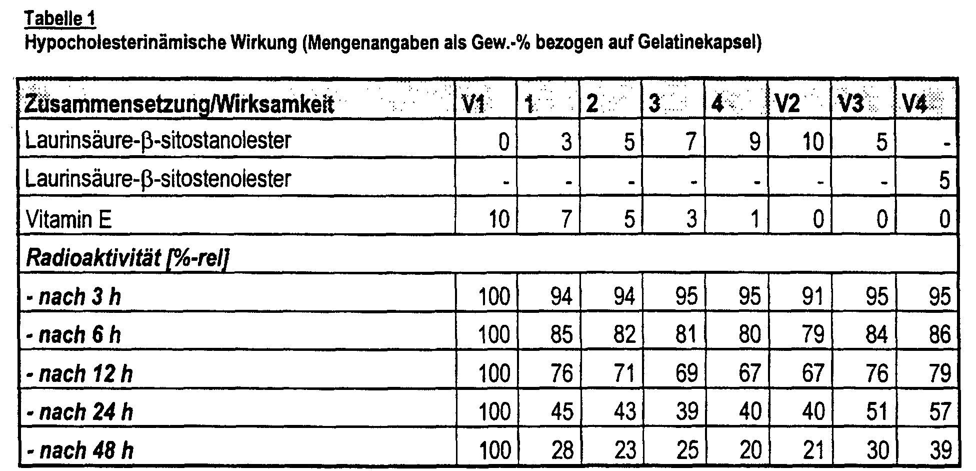

### Diskussion der Ergebnisse:

- Tocopherol alleine besitzt keine hypocholesterinämische Wirkung (Beispiel V1)
- Eine Mischung aus 9 Gew.-% Sitostanolester und 1 Gew.-% Tocopherol zeigt die gleiche Wirkung wie bei Einsatz von 10 Gew.-% Sitostanolester (Beispiele 4 und V2)
- Eine Mischung aus 5 Gew.-% Sitostanolester und 5 Gew.-% Tocopherol ist deutlich aktiver als 5 Gew.-% Sitostanolester alleine (Beispiele 2 und V3); sie ist vergleichbar wirksam wie 10 Gew.-% Sitostanolester alleine (Beispiel V2).

## Patentansprüche

1. Verwendung von Wirkstoffmischungen zur Herstellung von hypocholesterinämischen Mitteln, **dadurch gekennzeichnet, daß** man
(a) Phytostanolester und
(b) Tocopherole
einsetzt.

2. Verwendung nach Anspruch 1, **dadurch gekennzeichnet, daß** man als Komponente (a) β-Sitostanolester einsetzt.

3. Verwendung nach den Ansprüchen 1 und 2, **dadurch gekennzeichnet, daß** man als Komponente (a) Ester von β-Sitostanol mit Carbonsäuren der Formel **(I)** einsetzt,
**R**^{**1**}**CO-OH (I)**
in der R¹CO für einen aliphatischen, linearen oder verzweigten Acylrest mit 2 bis 22 Kohlenstoffatomen und 0 und/oder 1, 2 oder 3 Doppelbindungen steht.

4. Verwendung nach den Ansprüchen 1 bis 3, **dadurch gekennzeichnet, daß** man als Komponente (a) Ester von β-Sitostanol mit Fettsäuren mit 12 bis 18 Kohlenstoffatomen einsetzt.

5. Verwendung nach den Ansprüchen 1 bis 4, **dadurch gekennzeichnet, daß** man als Komponente (b) Tocopherole der Formel **(II)** einsetzt, 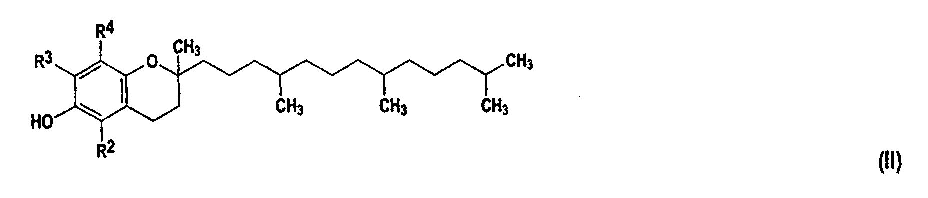 in der R², R³ und R⁴ unabhängig voneinander für Wasserstoff oder Methylgruppen stehen.

6. Verwendung nach den Ansprüchen 1 bis 4, **dadurch gekennzeichnet, daß** man als Komponente (b) α-Tocopherol (Vitamin E) einsetzt.

7. Verwendung nach den Ansprüchen 1 bis 6, **dadurch gekennzeichnet, daß** man als weitere Potenzierungsmittel Chitosane, Phytosterolsulfate und/oder (Desoxy-)Ribonucleinsäuren einsetzt.

8. Verwendung nach den Ansprüchen 1 bis 7, **dadurch gekennzeichnet, daß** man die Komponenten (a) und (b) im Gewichtsverhältnis 99 : 1 bis 1 : 99 einsetzt.

9. Verwendung nach den Ansprüchen 1 bis 8, **dadurch gekennzeichnet, daß** man die Komponenten (a) und (b) in Gelatine verkapselt.

10. Verwendung nach Anspruch 9, **dadurch gekennzeichnet, daß** man die Komponenten (a) und (b) jeweils in Mengen von 0,1 bis 50 Gew.-% - bezogen auf das Gewicht der Gelatinekapseln - einsetzt.

## Claims

1. The use of active-substance mixtures for the production of hypocholesterolemic preparations, **characterised in that** (a) phytostanol esters and (b) tocopherols are used.

2. The use claimed in claim 1, **characterised in that** β-sitostanol esters are used as component (a).

3. The use claimed in claims 1 and 2, **characterised in that** esters of β-sitostanol with carboxylic acids corresponding to formula (I):
R¹CO-OH (I)
in which R¹CO is an aliphatic, linear or branched alkyl group containing 2 to 22 carbon atoms and 0 and/or 1, 2 or 3 double bonds, are used as component (a).

4. The use claimed in claims 1 to 3, **characterised in that** esters of β-sitostanol with fatty acids containing 12 to 18 carbon atoms are used as component (a).

5. The use claimed in claims 1 to 4, **characterised in that** tocopherols corresponding to formula (II): 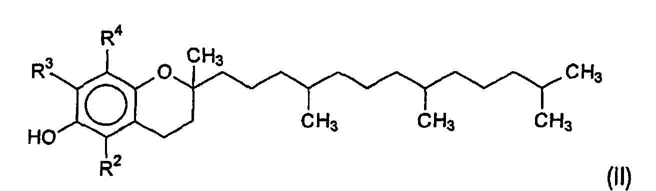 in which R², R³ and R⁴ independently of one another represent hydrogen or methyl groups, are used as component (b).

6. The use claimed in claims 1 to 4, **characterised in that** α-tocopherol (vitamin E) is used as component (b).

7. The use claimed in claims 1 to 6, **characterised in that** chitosans, phytosterol sulfates and/or (deoxy)ribonucleic acids are used as further potentiating agents.

8. The use claimed in claims 1 to 7, **characterised in that** components (a) and (b) are used in a ratio by weight of 99:1 to 1:99.

9. The use claimed in claims 1 to 8, **characterised in that** components (a) and (b) are encapsulated in gelatine.

10. The use claimed in claim 9, **characterised in that** components (a) and (b) are each used in quantities of 0.1 to 50% by weight, based on the weight of the gelatine capsules.

## Revendications

1. Utilisation de mélanges de substances actives pour la préparation d'agents hyperchoestérolémiants,
**caractérisée en ce qu'**
on utilise
(a) des esters de phytostanol et
(b) des tocophérols.

2. Utilisation selon la revendication 1,
**caractérisée en ce qu'**
on utilise comme composant (a) l'ester de β-sitostanol.

3. Utilisation selon les revendications 1 et 2,
**caractérisée en ce qu'**
on ulilise comme composant (a) des esters de β-sitostanol avec des acides carboxyliques de formule (I)
R¹CO-OH (I)
dans laquelle R¹CO représente un radical acyle aliphatique, linéaire ou ramifié comportant de 2 à 22 atomes de carbone et 0 et/ou 1, 2 ou 3 doubles liaisons.

4. Utilisation selon les revendications 1 à 3,
**caractérisée en ce qu'**
on utilise comme composant (a) des esters de β-sitostanol avec des acides gras comportant de 12 à 18 atomes de carbone.

5. Utilisation selon les revendications 1 à 4,
**caractérisée en ce qu'**
on utilise comme composant (b) des tocophérols de formule (II) 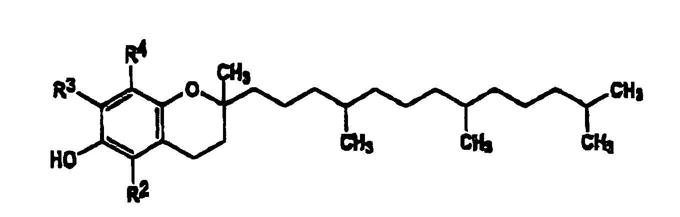 dans laquelle R², R³ et R⁴ représentent indépendamment l'un de l'autre un hydrogène ou des groupes méthyle.

6. Utilisation selon les revendications 1 à 4,
**caractérisée en ce qu'**
on utilise comme composant (b) d'α-tocophérol (vitamine E).

7. Utilisation selon les revendications 1 à 6,
**caractérisée en ce qu'**
on utilise comme autre agent de potentialisation les chitosanes, les sulfates de phytostérol et/ou des acides (désoxy) ribonucléiques.

8. Utilisation selon les revendications 1 à 7,
**caractérisée en ce qu'**
on utilise les composants (a) et (b) dans un rapport pondéral de 99:1 à 1:99.

9. Utilisation selon les revendications 1 à 8,
**caractérisée en ce qu'**
on encapsule les composants (a) et (b) dans de la gélatine.

10. Utilisation selon la revendication 9,
**caractérisée en ce qu'**
on utilise à chaque fois les composants (a) et (b) à des quantités de 0,1 à 50 % en poids - par rapport poids des gélules de gélatine.
